# FASCICULE DE BREVET EUROPEEN

(11) **EP 3 622 053 B1**
(45) Date de publication et mention de la délivrance du brevet: **09.02.2022**
(21) Numéro de dépôt: 18720323.7
(22) Date de dépôt: 11.04.2018
(51) Int. Cl.: C12N 5/00

(54) **UTILISATION D'ACIDE URIQUE POUR UNE CULTURE CELLULAIRE SOUS ATMOSPHERE AEROBIE NON SUPPLEMENTEE EN CO2**
VERWENDUNG VON HARNSÄURE FÜR EINE ZELLKULTUR IN EINER NICHT MIT CO2 ERGÄNZTEN AEROBEN ATMOSPHÄRE
USE OF URIC ACID FOR A CELL CULTURE IN AN AEROBIC ATMOSPHERE NOT SUPPLEMENTED WITH CO2

(30) Priorité: 18.04.2017 FR 1753329
(43) Date de publication de la demande: 18.03.2020
(73) Titulaire: Fondation Méditerranée Infection, 13005 Marseille (FR); Université d'Aix Marseille, 13284 Marseille Cedex 07 (FR); Assistance Publique - Hôpitaux de Marseille, 13005 Marseille (FR); Centre National de la Recherche Scientifique (CNRS), 75016 Paris (FR); INSERM (Institut National de la Santé et de la Recherche Médicale), 75013 Paris (FR)
(72) Inventeur: RAOULT, Didier, 13008 Marseille (FR); LA SCOLA, Bernard, 13109 Simiane-Collongue (FR)
(74) Mandataire: Cabinet Beau de Loménie
(86) Numéro de dépôt international: PCT/FR2018/050914
(87) Numéro de publication internationale: WO 2018/193186

(56) Documents cités:
- WO-A1-2015/162366
- WO-A1-2015/162377
- FR-A1- 3 020 378
- Anonymous: "Uric Acid in Cell Culture", , 4 juin 2013 (2013-06-04), XP055177496, Extrait de l'Internet: URL:https://web.archive.org/web/2013060410 2234/http://www.sigmaaldrich.com/life-scie nce/cell-culture/learning-center/media-exp ert/uric-acid.html [extrait le 2015-03-18]
- N. DIONE ET AL: "A quasi-universal medium to break the aerobic/anaerobic bacterial culture dichotomy in clinical microbiology", CLINICAL MICROBIOLOGY AND INFECTION., vol. 22, no. 1, 1 janvier 2016 (2016-01-01), pages 53-58, XP055361668, United Kingdom, Switzerland ISSN: 1198-743X, DOI: 10.1016/j.cmi.2015.10.032

## Description

La présente invention concerne des milieux et procédés de culture cellulaire ou de culture intracellulaire de microorganismes et notamment la supplémentation des milieux de culture cellulaire classiques pour la culture cellulaire ou la culture intracellulaire de microorganismes permettant de mettre en œuvre une culture sous atmosphère non supplémentée en CO₂.

La présente invention concerne plus particulièrement des milieux et procédés de culture autorisant la culture de lignée de cellules eucaryotes d'organisme pluricellulaire et la propagation intracellulaire de microorganismes dans des dites cellules eucaryotes d'organisme pluricellulaire, notamment de bactéries intracellulaires, de virus ou parasites qui s'y multiplient en l'absence de CO₂.

Les cultures cellulaires, que cela soit pour la culture de cellules eucaryotes seules ou utilisées comme support pour la culture et l'isolement de microorganismes, notamment de bactéries intracellulaires strictes et de virus, nécessitent souvent l'utilisation d'une atmosphère contenant du CO₂, notamment dans de l'air contenant 5% de CO₂. L'objectif est, par la présence de CO₂ dans l'atmosphère de culture, de tamponner le milieu et équilibrer le pH en compensant l'augmentation de l'acidité dans le milieu de culture (production de H⁺) résultant du métabolisme cellulaire avec la production de HCO3⁻. Cette augmentation d'acidité conduit autrement (en absence de CO₂) à une mortalité des cellules. Cette supplémentation en CO₂ est surtout nécessaire les 24 premières heures. Ensuite si la boite de culture est fermée hermétiquement ce besoin disparait.

La culture de certains microorganismes tels que parasites tel que du genre Plasmodium nécessite en sus du CO₂ l'utilisation d'azote ou autre gaz inerte visant à abaisser davantage encore le taux d'oxygène à 10% ce qui nécessite par exemple l'utilisation d'étuves trigaz (N₂ 85%, O₂ 10%, CO₂ 5%).

La mise en œuvre de CO₂ pour ce type de culture cellulaire présente les inconvénients suivants.

Tout d'abord, cela requière une installation couteuse en termes de dispositifs divers à utiliser (tuyaux, manodétendeurs, alarmes, enceintes d'incubateur spéciales, voire une étuve trigaz) ainsi que la consommation de fluide gazeux, le CO₂ en bouteille représentant un coût consommable non négligeable.

De surcroit, lorsque cette production de cellules est utilisée pour l'isolement et la propagation de microorganismes dangereux exigeant des niveaux de sécurité élevés, notamment pour des bactéries intracellulaires et virus de classe 3, se pose le problème de l'utilisation du CO₂ dans un laboratoire de classe NSB3. Dans ces laboratoires, il n'est pas possible de faire entrer les fûts de CO₂ et donc il est nécessaire de déployer un système de canalisations amenant le CO₂ à l'intérieur depuis l'extérieur. Par ailleurs, une fuite de CO₂ dans un laboratoire confiné présente des risques. En cas de fuite, le taux d'oxygène diminue avec risque d'asphyxie pour le personnel.

Dans tous les cas, cela justifie l'installation de détecteurs de CO₂ branchés sur alarmes.

Enfin, dans des zones mal équipées comme des laboratoires de campagne en expédition de terrain, l'utilisation de CO₂ est impossible tout autant que les matériels tels qu'étuves trigaz.

Le but de la présente invention est de fournir des conditions améliorées alternatives de culture cellulaire permettant de se passer de la mise en œuvre d'une culture cellulaire sous atmosphère CO₂ et ainsi éviter toutes les contraintes de matériels mentionnées ci-dessus pour cultiver des cellules et des microorganismes tel que bactéries intracellulaires, virus et parasites qui se multiplient à l'intérieur des dites cellules le cas échéant.

On a décrit dans WO 2014/064359 d'améliorer et faciliter les conditions de croissance en culture acellulaire des bactéries dont la croissance est sensible à la tension en oxygène et notamment des bactéries qui tolèrent mal des tensions élevées d'oxygène et pour lesquelles une croissance optimale de la dite bactérie requière une atmosphère d'incubation à teneur en oxygène relativement réduite par rapport à la teneur en oxygène de l'air, lesdites bactéries étant choisies parmi les bactéries anaérobies, y compris les bactéries anaérobies strictes, et les bactéries microaérophiles qui ne sont capables de cultiver que sous une atmosphère comprenant une concentration d'oxygène diminuée, le plus communément à environ 2-2,5%.

Dans WO 2014/064359, on a proposé d'ajouter certains composés antioxydants à savoir l'acide ascorbique, le glutathion et l'hydrosulfure de sodium dans un milieu de culture acellulaire pouvait permettre d'améliorer la croissance des dites bactéries et/ou de cultiver des dites bactéries en présence de quantités plus élevées d'oxygène.

Dans WO 2014/ 064359, on fournit donc un procédé de culture in vitro de bactéries en milieu de culture acellulaire, de telles bactéries anaérobies ou microaérophiles intracellulaires, caractérisé en ce que l'on ajoute dans le dit milieu de culture acellulaire un composé antioxydant choisi parmi l'acide ascorbique, le glutathion et l'hydrosulfure de sodium, et l'on cultive la dite bactérie dans ledit milieu de culture en présence d'oxygène.

Dans certaines conditions l'acide ascorbique apparait toxique et/ou trop acide vis à vis de certaines bactéries et/ou pour certain milieux de culture à fortes doses. Ceci a été mis en évidence notamment pour *Mycobacterium tubercu*/*osis* (1). D'autre part, le glutathion est très onéreux.

Dans WO 2015/162377, il a été décrit qu'un effet équivalent sur la croissance des dites bactéries anaérobies ou bactéries microaérophiles sensibles à l'oxygène pouvait également être obtenu en ajoutant dans le dit milieu de culture de l'acide urique (7,9-dihydro-1H-purine-2,6,8(3H)-trione ou 2,6,8-trioxypurine) à la place des composés antioxydants décrits dans WO 2014/064259.

L'acide urique, bien qu'ayant dans certaines conditions des propriétés antioxydantes, n'est pas classiquement utilisé en microbiologie à titre de composé antioxydant car dans certaines conditions il présente au contraire des propriétés oxydantes (2).

Selon la présente invention, on a découvert que l'ajout d'acide urique à forte dose dans un milieu de culture cellulaire permet d'éviter la mise en œuvre d'une atmosphère de culture contenant du CO₂. L'acide urique a des capacités de type anti-oxydantes mais on ne pouvait prévoir qu'il pourrait jouer le rôle de tampon du milieu de culture cellulaire généralement dévolu au CO₂. Il est possible qu'il protège les cellules d'un effet délétère de dérivés de l'oxygène, le temps que par leur activité propre de détoxification les cellules tamponnent elle-même le milieu. Cet effet non totalement élucidé ne pouvait pas être déduit de l'état de l'Art.

Plus précisément, la présente invention fournit un procédé de culture de lignée de cellules eucaryotes d'organisme pluricellulaire in vitro caractérisé en ce que l'on ajoute dans le milieu de culture cellulaire de l'acide urique à une concentration d'au moins 0.1 g/L et on réalise la culture sous une atmosphère d'air ambiant non supplémentée en CO₂, la multiplication des dites cellules étant apte à être améliorée par culture sous atmosphère supplémentée en CO₂, notamment dans de l'air enrichi à 5% de CO₂ notamment au moins dans les premières 24 heures, en l'absence d'acide urique dans le dit milieu de culture.

Plus particulièrement, on réalise une culture intracellulaire de microorganismes dans des dites cellules en dit milieu de culture cellulaire contenant de l'acide urique à une concentration d'au moins 0.1 g/L sous une atmosphère d'air ambiant non supplémentée en CO₂.

De préférence, l'acide urique est mis en œuvre à une concentration de 0,4 à 1 g/L.

Plus particulièrement, on réalise une culture intracellulaire d'un microorganisme choisi parmi les bactéries intracellulaires strictes, les virus et les parasites.

Plus particulièrement, on réalise une culture intracellulaire d'un dit microorganisme dans un récipient ouvert sous une atmosphère d'air ambiant non supplémenté en CO₂ et on ferme le récipient après 24h de culture.

Dans un mode de réalisation, on réalise une culture intracellulaire d'une bactérie intracellulaire stricte choisie parmi les genres *Rickettsia, Orientia, Lawsonia, Coxiella, Ehrlichia, Chlamydia, Chlamydiophila, Anaplasma* et *Neorickettsia.* Il s'agit là de bactéries intracellulaires strictes d'intérêt médical et vétérinaire.

Plus particulièrement encore, on réalise une culture intracellulaire d'une bactérie intracellulaire stricte choisie parmi *Coxiella burnetii* et une espèce *Rickettsia sp.*

Dans un autre mode de réalisation, on réalise une culture intracellulaire d'un virus choisi parmi les familles *Poxviridae, Herpesviridae, Adenoviridae, Papillomaviridae, Polyomaviridae, Parvoviridae, Picornaviridae, Caliciviridae, Astroviridae, Flaviviridae, Togaviridae, Coronaviridae, Paramyxoviridae, Rhabdoviridae, Filoviridae, Orthomyxoviridae, Arenaviridae, Bunyaviridae, Reoviridae, Hepadnaviridae* et *Retroviridae.* Il s'agit là de virus d'intérêt médical et vétérinaire.

Dans un autre mode de réalisation, on réalise une culture d'un parasite choisi parmi *Plasmodium, Apicomplexa* et *Leishmania*.Plus particulièrement encore, le dit parasite est *Plasmodium falciparum.*

Plus particulièrement encore, on réalise une culture de cellules choisies parmi les lignées 3T6, 3T3, BA886, BHK21, Caco-2, CE, CLC, CRL1439, CRL1442, DF1, DH82, EC2, EC53, HEL, Hela, Hep2, HL60, HT29, J774, Jurkat, L929, Mac Coy, MRC5, MSDHP, P388, Raw264, Tatoo, TF1, THP1, U937, VERO, MDCK, BGM, MA104, ViroE6 et CLNh11.

Ces cellules sont appropriées pour la culture intracellulaire des dits microorganismes excepté les parasites du genre Plasmodium pour lesquels on utilise des érythrocytes humains.

Plus particulièrement encore, les cellules sont choisies parmi les cellules de fibroblastes humains de la lignée HEL, les cellules fibroblastiques de souris de la lignée L929, des cellules de reins de singe de la lignée VERO et les cellules de type fibroblastes de la lignée MRC5, notamment pour réaliser la culture d'une bactérie intracellulaire ou un virus.

Plus particulièrement encore, les dites cellules sont des érythrocytes humains (globules rouges) pour réaliser la culture intracellulaire d'un parasite du genre *Plasmodium, notamment Plasmodium falciparum* dans un milieu de culture contenant de l'acide urique sous une atmosphère non supplémentée en CO₂ et non diminuée en O₂. En revanche, en l'absence d'acide urique, la multiplication de *Plasmodium falciparum* requiert une atmosphère supplémentée en CO₂ et diminuée en oxygène, notamment une atmopshère trigaz 10%O₂, 85%N₂ et 5%CO₂, en l'absence d'acide urique dans le dit milieu de culture.

La présente invention fournir également un milieu de culture utile pour un procédé selon l'invention caractérisé en ce qu'il comprend un milieu de culture de base pour culture cellulaire, le dit milieu de culture de base étant enrichi en acide urique, le dit milieu de culture un milieu de culture de base pour culture cellulaire, le dit milieu de culture de base étant enrichi en acide urique à une concentration d'acide urique d'au moins 0,1 g/L, le dit milieu de culture de base étant apte à permettre la culture des dites cellules eucaryotes par culture sous atmosphère supplémentée en CO₂, de préférence dans de l'air enrichi à 5% de CO₂, en l'absence d'acide urique dans le dit milieu de culture de base.

De préférence, le milieu de culture selon l'invention comprend une concentration d'acide urique de 0,4 à 1 g/L.

Dans un mode de réalisation, le dit milieu de culture de base est un milieu de culture cellulaire comprenant au moins les composants suivants :
- une pluralité des sels inorganiques choisis notamment parmi les chlorure de calcium, nitrate de calcium, nitrate ferrique, sulfate de magnésium, chlorure de potassium, bicarbonate de sodium, chlorure de sodium et phosphate de sodium, de préférence au moins 4 dits sels inorganiques;
- une pluralité d'acides aminés choisis notamment parmi les arginine, cystine, glycine, histidine, isoleucine, leucine, lysine, méthionine, phénylalanine, serine, thréonine, tryptophane, tyrosine, valine, asparagine, aspartate, glutamine, acide glutamique, proline, sérine et valine, de préférence l'ensemble des dits acides aminés;
- une pluralité de vitamines choisies notamment parmi les choline, acide folique, inositol, niacinamide, nicotinamide), acide pantothénique, pyridoxal, pyridoxine, riboflavine, thiamine, biotine, acide aminobenzoique et cobalamine, de préférence au moins 3 dites vitamines ; et
- une source d'hydrate de carbone, de préférence un sucre, notamment du glucose.
DMEM (« Dubelcco Modified Eagle Médium » ), RPMI (« Roswell Park Memorial Institute medium ») ou IMDM (« Iscove's Modified Dulbecco's Media ») ainsi que toutes leurs variantes

Plus particulièrement, l'acide urique est mis en œuvre sans composé antioxydant additionnel dans le dit milieu de culture.

D'autre caractéristiques et avantages de l'invention apparaitront à la lumière de la description détaillée des exemples de réalisations suivants se référant aux figures suivantes dans lesquelles :

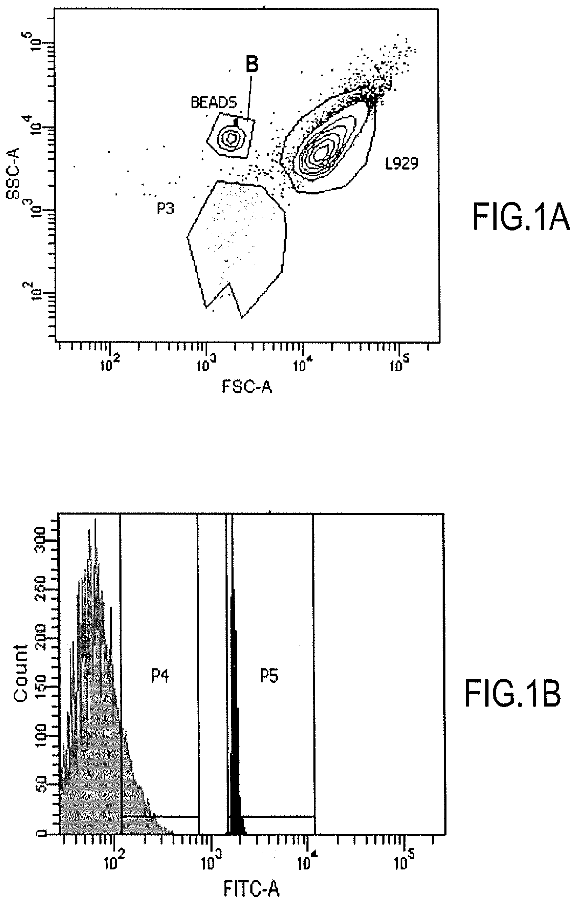

- les Figures 1A et 1B représentent la quantification par cytométrie de flux avec le fenêtrage utilisé pour les cellules L929 et les billes fluorescentes (« B »). (Nombre de cellules /nombre de billes) × concentration de billes (1053 billes/µl) × facteur de dilution. La figure 1A représente les éléments comptés en taille structure (1 nuage billes et 1 nuage cellules) et la figure 1B représente la fluorescence des éléments comptés (billes en P5 et cellules en P4) ;

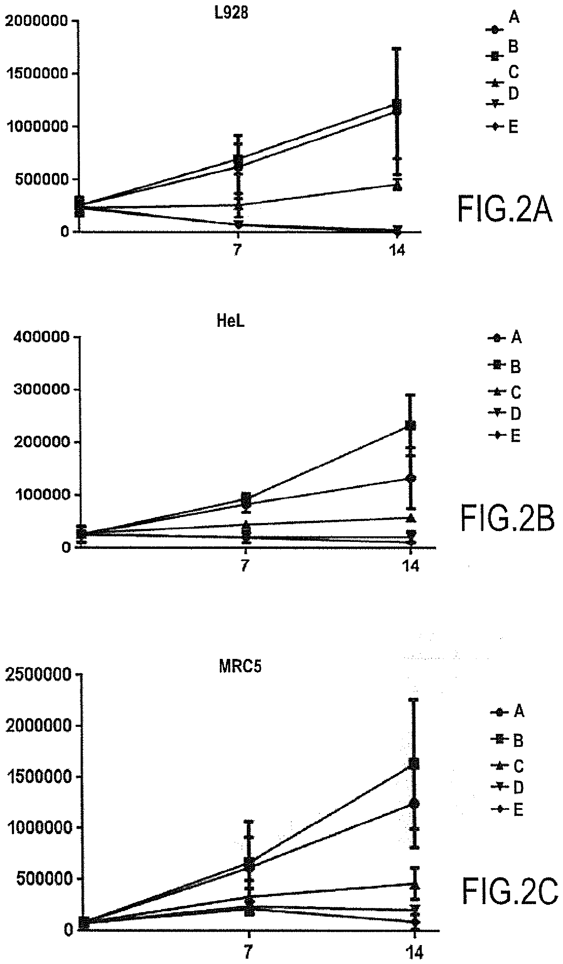

- les figures 2A, 2B et 2C représentent le comptage de cellules en ordonnée vs la durée de culture en abscisses (en jours) pour la culture des cellules L929 (figure 2A), MRC5 (figure 2B), ou HeL (figure 2C) dans différentes conditions de culture A à E explicitées ci-après ;

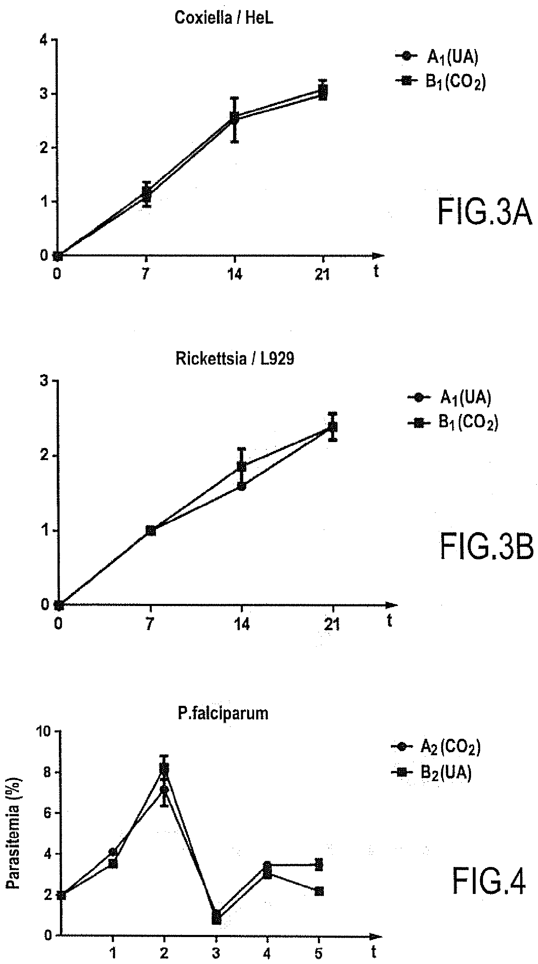

- les figure 3A et 3B représentent les croissances de *Coxiella burnetii* sur cellules HeL (figure 3A) et *Rickettsia conorii* sur la lignée cellulaire L929 (figure 3B) dans deux conditions : « UA »= présence d'acide urique dans le milieu sans CO₂ dans l'atmosphère et « CO₂ » = absence de d'acide urique dans le milieu et présence de CO₂ dans l'atmosphère;
- la figure 4 représente la croissance de *Plasmodium falciparum* sur les globules rouges A⁺ dans deux conditions, avec de l'acide urique dans des flacons fermés (courbe « B2 (UA) ») et sans acide urique dans des conditions de croissance standard (10% O₂, 5% CO₂ et 85% N₂) (courbe « A2 (CO₂) »); et

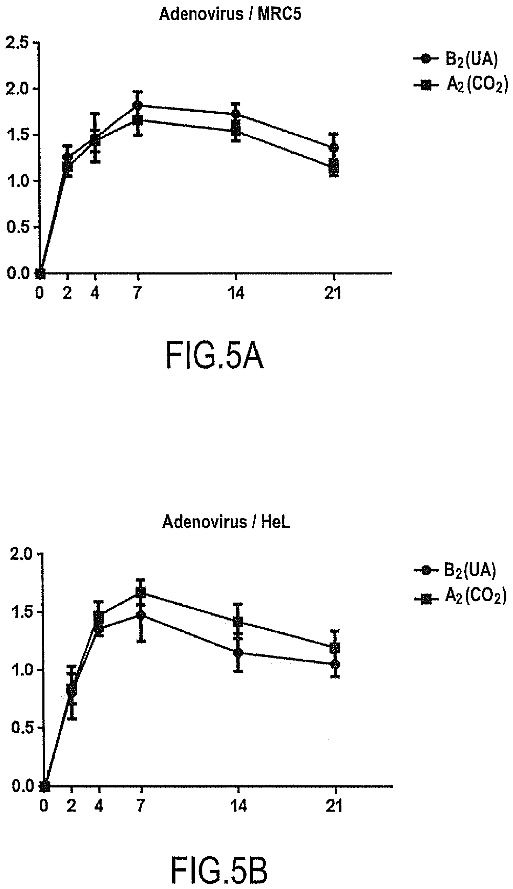

- les figures 5A et 5B représentent les croissances d'Adenovirus sur des cellules MRC5 (figure 5A) et HeL (figure 5B) dans deux conditions, avec de l'acide urique dans des flasques fermées (courbe « B2 (UA) ») et sans acide urique dans des conditions de croissance standard avec 5% de CO₂ (courbe « A2 (CO₂) »), la valeur p >0,05.

Des lignées cellulaires de fibroblaste murin L929 et de fibroblastes humains HEL et MRC5 ont été utilisées. Ce sont les supports d'hôtes cellulaires nécessitant du CO₂.

L'étude a été menée en utilisant deux conditions de culture suivantes.
1) Premièrement, des conditions de routine avec 5% de CO₂ dans de l'air où toutes les lignées cellulaires ont été maintenues dans des flasques de culture cellulaire de 75 cm2 à 37°C dans du milieu DMEM (# 11960-069, Invitrogen Carlsbad, CA) supplémenté avec 10% de sérum bovin fœtal inactivé par la chaleur (à 56°C pendant 30 minutes) sous 5% de CO₂ avant fermeture du flacon au bout de 24h.
2) La seconde condition comprenait le même milieu de base mais supplémenté par l'acide urique, préparé comme suit.

L'acide urique (SIGMA-Aldrich, Life Science U2625-100G, St. Quentin Fallavier Cedex 38297 France), à une concentration de 10 g dans 1 L de solution tampon PBS filtrée à 0,22 µm, pH ajusté à 7.

La concentration d'acide urique dans le milieu final était de 2393,6 µM (403,2 mg/L), contrôlée et quantifiée en utilisant la technique enzymatique de l'uricase sur un analyseur modulaire Cobas 8000 (Roche diagnostics Indianapolis, USA).

Dans le milieu complémenté en acide urique, les flacons ont été incubés à 37°C sans CO₂, flacon fermé.

Pour les microorganismes, on a utilisé *Coxiella burnetii* (souche Nine mile II) pour infecter des cellules HEL et *Rickettsia conorii* (souche Seven) pour infecter des cellules L929.

Pour les virus, on a utilisé un adénovirus de type C (souche MRS2008 1365148) qui infecte les cellules HEL et MRC5.

On a infecté chaque cellule avec son microorganisme correspondant à une multiplicité d'infection (MOI) définie dans les deux conditions de culture précédemment citées.

Avec les parasites, on a maintenu des parasites de *Plasmodium falciparum* (clone W2 résistant à la chloroquine d'Indochine, BEI Resources, NIAID, NIH, Virginie, USA, Ref. MRA-157) en culture continue dans du milieu RPMI 1640 (Invitrogen, Carlsbad, CA), supplémenté avec 10% de sérum humain (Abcys SA Paris, France) et tamponné avec du HEPES 25 mM et du NaHCO3 25 mM.

Les parasites ont été cultivés dans des globules rouges humains de type A⁺ dans des conditions atmosphériques contrôlées qui consistaient en 10% d'O₂, 5% de CO₂ et 85% de N2 à 37°C. Le milieu a été changé tous les jours et la parasitémie a été contrôlée par examen au microscope des frottis sanguins avec un kit RAL^{®} 555 (RAL Diagnostics, Martillac, France). Lorsque les parasites atteignent les stades d'anneau, la parasitémie est ajustée à 2% et deux conditions sont testées: le milieu habituel dans des flasques de culture cellulaire de 25 cm2 et l'acide urique ajouté au milieu à la même concentration dans 25 cm2 de culture cellulaire Standard fermé.

Les essais ont été effectués en triplicat. On a surveillé l'absence de contamination chaque semaine en contrôlant tous les milieux de culture utilisés pour toute croissance bactérienne sur des milieux de culture gélosés.

### A) Quantification, collecte de données et mesure de la croissance cellulaire.

Les taux de croissance et les densités cellulaires finales ont été quantifiés par cytométrie de flux en utilisant une suspension de billes fluorescentes (Cytocount TM) comme population de référence. Le nombre absolu de cellules (cellules/µl) dans chaque condition de culture a été calculé en utilisant l'équation suivante: (Nombre de cellules comptées/Nombre de billes comptées)× concentration en billes (1054 billes/µl) × Facteur de dilution (Les billes dont on connait la concentration servent d'étalons pour le comptage absolu des cellules) [5]. Les paramètres ont été ajustés en utilisant des concentrations diluées des différents types cellulaires avec et sans billes. L'acquisition des données a été réalisée en fonction des paramètres de taille et de structure (FSC (diffusion directe) et SSC (dispersion latérale) afin de définir un portage fixe spécifique à chaque type de cellule.

On a également utilisé le fluorochrome Sytox Dead Cell Stain TM qui ne marque et ne colore que les cellules mortes, pour la cytométrie en flux (Molecular probes, life technologies USA) pour évaluer la viabilité des cellules. Après une période d'incubation appropriée, les cellules ont été décollées en utilisant 3 ml de trypsine pendant 5 minutes à 37°C puis remises en suspension dans leur milieu, le Sytox a été ajouté et les échantillons ont été incubés pendant 10 minutes à température ambiante dans l'obscurité. Puis 25 µl de billes ont été ajoutées avant le passage des échantillons sur le cytomètre. Le nombre total d'événements enregistrés était de 20 000 pour le comptage cellulaire en utilisant un analyseur BD LSR Fortessa ^{™} Cell Analyser (Société BD Bioscience). Les données ont été analysées à l'aide du logiciel BD FACSDiva 6.2 (Société BD Bioscience).

Le nombre d'événements a été calculé, et on a calculé le nombre de cellules dans 1 ml d'échantillon en suivant l'équation détaillée ci-dessus et ci-après. Des quantifications ont été effectuées aux jours J=0, 7 et 14, après inoculation.

La Figure 1 représente la quantification par cytométrie de flux avec le gating utilisé pour les cellules L929 et les billes fluorescentes. (Nombre de cellules /nombre de billes) × concentration de billes (1053 billes/µl) × facteur de dilution. Sur les figures 1Aet 1B, les valeurs sont pour un tube fermé avec de l'acide urique (« UA ») à J=7 jours.

Sur la figure 1A, l'abscisse représente les propriétés optiques reflétant la taille des cellules, P3 représente les débris cellulaires à exclure du comptage et l'ordonnée représente les propriétés reflétant la structure et la granulométrie des cellules, P4 représente l'auto fluorescence des cellules et P5 représente le pic de fluorescence des billes.

On voit que l'on peut discriminer billes et cellules permettant d'avoir un compte absolu de chaque population (billes et cellules).

### B) Quantification, collecte de données et mesure des bactéries intracellulaires et croissance de virus.

On a répété les mêmes tests décrits ci-dessus après avoir infecté les cellules avec deux bactéries intracellulaires différentes: *Coxiella burnetii* (souche Nine mile II) pour une culture avec les cellules HEL, *Rickettsia conorii* (souche Seven) pour une culture avec les cellules L929 et Adenovirus pour une culture avec les cellules HEL ou MRC5.

Dans ces tests, la croissance bactérienne a été quantifiée en utilisant qPCR ciblant des gènes spécifiques: IS11 pour *C. burnetii,* R.co7 gen hypothical protein pour *R. conorii* (3,4,6) et le gène VTB2 pour l'adénovirus (7).

Les valeurs de Ct de la PCR ont été corrélées à la charge bactérienne quantifiée par cytométrie de flux dans les conditions de CO₂ et d'acide urique et par la technique de dilution de point final TCID50 pour l'adénovirus. Les quantifications ont été effectuées au jour 0, 7, 14 et 21 post-inoculation.

### C) Quantification de la croissance des parasites

La croissance des parasites a été estimée par examen microscopique des frottis sanguins après coloration avec le kit RAL^{®} 555. L'évolution de la croissance parasitaire a été surveillée pendant 5 jours après le début des dosages dans les deux conditions. Aux jours 2 et 4, les parasites ont été dilués à 1/10 et 1/5 respectivement pour prévenir la mort du parasite due à une parasitémie élevée.

### D) Analyses statistiques

D'abord, des tests statistiques ont été effectués afin de montrer le besoin critique des cellules en CO₂ pour la croissance. On a utilisé un test de Mann-Whitney pour comparer le nombre moyen de cellules pour chaque lignée cellulaire de l'état de contrôle négatif (sans CO₂ et sans acide urique) et la condition de routine avec 5% de CO₂. Un autre essai a été effectué en comparant le nombre moyen de cellules entre la condition avec du CO₂ à 5% et le CO₂ dans un ballon ouvert. (Une valeur p <0,05 représente une différence significative).

Pour la comparaison entre l'acide urique et le CO₂, on a utilisé le nombre moyen de cellules et la croissance bactérienne / virale / parasitaire dans les deux conditions de culture en utilisant un test de Mann-Whitney (le nombre de cellules obtenu avec les deux conditions de culture était considéré comme significativement différent si la valeur p <0,05).

### E) Résultats

On a comparé la culture couramment utilisée dans les conditions atmosphériques de CO₂ aux nouvelles conditions de culture avec de l'acide urique.

Plus précisément, sur les figures 2A-2C :
- A correspond à une culture en présence d'acide urique sous atmosphère aérobie et sans CO₂ en flacon fermé ;
- B correspond à une culture en absence d'acide urique sous atmosphère d'air contenant 5% de CO₂ en flacon fermé après 24h ;
- C correspond à une culture en absence d'acide urique sous atmosphère aérobie et sans CO₂ en flacon fermé après 24h ;
- D correspond à une culture en absence d'acide urique sous atmosphère d'air contenant 5% de CO₂ en flacon ouvert ;
- E correspond à une culture en présence d'acide urique sous atmosphère aérobie en flacon ouvert.

La quantification cellulaire a été effectuée par cytométrie de flux. Au jour 0, la comparaison moyenne du nombre de cellules entre la condition habituelle de 5% de CO₂ (courbe B) et le témoin négatif (sans CO₂ et sans acide urique courbe C pour les trois lignées cellulaires L929, HEL et MRC5 n'a montré aucune différence significative. Aux jours 7 et 14, seules les conditions avec 5% de CO₂ (cellules exposées à 5% de CO₂ pendant 24h puis flasque fermée) (courbe B) montrent une meilleure croissance cellulaire par rapport aux conditions sans CO₂ ou 5% de CO₂ flasque ouverte en continu (courbe D), sans CO₂ et sans acide urique flasque fermée (courbe C) (p <0,05), ce qui confirme que le CO₂ est essentiel à la croissance cellulaire normale. L'acide urique était efficace pour maintenir les lignées cellulaires testées dans de bonnes conditions de culture avec une croissance correcte et de bonnes capacités de multiplication par rapport à la croissance obtenue dans des conditions de routine exposées au CO₂ (courbe B) à condition de garder les flasques fermées car si elles sont ouvertes l'effet est perdu.

Le taux de croissance pour les lignées cellulaires L929, HEL et MRC5 était similaire dans les deux conditions de culture, sans différence significative (p-valeur = 0,709).

Sur les figures 2A, 2B et 2C, on ne relève aucune différence significative de croissance détectée (p-value > 0.05) entre la culture des cellules L929 (figure 2A), MRC5 (figure 2B), ou HeL (figure 2C) pour les deux conditions de culture : seules les cellules cultivées avec 5% de CO₂ flasque fermée après 24h et acide urique flasque fermée arrivent à croitre (respectivement courbes B et A). Pour toutes les autres conditions testées (CO₂ flasque ouverte en continu, sans CO₂ flasque fermée, acide urique flasque ouverte respectivement courbes D, C et E), aucune croissance cellulaire n'est observée.

On peut observer la croissance continue des lignées cellulaires confirmée par les valeurs aux jours 7 et 14. Toutes les lignées cellulaires testées ont été suivies par des observations microscopiques avant quantification. Aucune modification ou anomalie de la forme ou de l'adhérence des cellules n'a été détectée dans les conditions de culture en présence d'acide urique pendant toute la période d'essai (courbes A) La sub-culture des cellules a été maintenue en routine dans le laboratoire. Aucune contamination n'a été détectée dans aucun des tests. Pour les cultures en flacons ouverts (courbes D et E), plusieurs agrégats ont été détectés par cytométrie de flux ou sous microscope optique et les cellules étaient presque mortes et incapables de croître. En outre, aucune anomalie de la forme cellulaire ou de l'adhérence n'a été observée et on a seulement détecté une croissance limitée des cellules dans des conditions dépourvues de CO₂ et d'acide urique (courbes C).

La croissance des microorganismes a été contrôlée par qPCR. La croissance de *C. burnetii* sur cellules HEL, *R. conorii* sur la lignée cellulaire L929 et de *P. falciparum* sur les érythrocytes était presque aux mêmes niveaux dans les deux conditions de culture et aucune différence significative n'a été observée entre les deux (respectivement p-valeur = 0,861, 0,683, 0,731) (Figures 3A-3B et 4A-4B).

Les figure 3A et 3B représente la croissance de *Coxiella burnetii* sur cellules HeL (figure 3A) cultivée dans les deux conditions, et la croissance de *Rickettsia conorii* sur la lignée cellulaire L929 (figure 3B) en présence d'acide urique en culture et en culture de routine avec CO₂, avec la valeur p >0,05 (courbe «A1( UA) »= présence d'acide urique dans le milieu sans CO₂ dans l'atmosphère et courbe « B1 (CO₂) » = absence d'acide urique dans le milieu et présence de CO₂ dans l'atmosphère). L'axe X correspond aux temps de quantification en jours. L'axe Y correspond au log de la charge bactérienne (les valeurs logarithmiques sont obtenues après conversion des valeurs du seuil de Cycle (Ct.) Sur la base des courbes standards réalisées avec dilution sérielle 1: 10).

Les figures 4A et 4B représente la croissance de *Plasmodium falciparum* sur les globules rouges A⁺ («parasitémie en axe Y) dans les deux conditions, avec de l'acide urique dans des flacons fermés (courbe « B2 (UA) ») et sans acide urique dans des conditions de croissance standard (10% O₂, 5% CO₂ et 85% N2) (courbe « A2 (CO₂) ») avec une valeur p >0,05. La diminution après chaque point de temps (en jours sur l'axe des ordonnées) est due à la dilution du parasite avant de procéder à l'infection suivante.

En ce qui concerne *C. burnetii* et *R. conorii,* tous les titrages bactériens étaient normaux et les bactéries pouvaient se multiplier normalement dans les deux conditions. Comme on l'a observé sur les courbes de multiplication des bactéries testées sur les figures 3A-3B, les courbes en augmentation continue à chaque instant de l'infection attestent les taux de multiplication bactérienne.

Pour les parasites, les courbes figure 4 montrent une croissance continue pendant 48 heures après l'infection. La diminution après ce temps est due à la dilution du parasite avant de procéder à la prochaine infection. Après la dilution, la croissance du parasite redémarre et augmente normalement.

Sur les figures 5A et 5B, la croissance de l'adénovirus était similaire dans les deux conditions sur les deux lignées cellulaires, et aucune altération du cycle n'a pu être détectée. Les figures 5A et 5B représentent les croissances d'Adenovirus sur des cellules MRC5 (figure 5A) et HeL (figure 5B) dans deux conditions, avec de l'acide urique dans des flasques fermées (courbe « B2 (UA) ») et sans acide urique dans des conditions de croissance standard 5% de CO₂ (courbe « A2 (CO₂) »), la valeur p >0,05. L'axe X correspond aux temps de quantification en jours. L'axe Y correspond au logarithme de la charge virale (les valeurs logarithmiques sont obtenues après conversion des valeurs du seuil Cycle (Ct.) sur la base des courbes standards réalisées avec dilution série 1: 10).

Toutes les PCR effectuées trois fois ont montré une aptitude virale optimale dans les deux conditions de culture. Les courbes virales de multiplication ont montré des augmentations typiques de la charge virale à partir du jour 0, atteignant leur valeur maximale au jour 7. Les titres viraux ont commencé une augmentation lente après le jour 7, tendant vers un plateau entre le jour 7 et le jour 14 et atteignant la valeur la plus faible au jour 21.

### F) Conclusion.

Pour toutes les lignées cellulaires testées, une incubation initiale sous 5% CO₂ a pu être remplacée par l'ajout d'acide urique dans le milieu de culture à une concentration d'au moins 0.4 g/L en air ambiant. Les cellules et microorganismes se sont multipliés sans altération physiologique de forme ou d'adhésion.

### Références bibliographiques

[1] Vilchèze Catherine, Travis Hartman, Brian Weinrick, and William R. Jacobs Jr∗in Nat Commun. 2013; 4: 1881. doi:10.1038/ncomms2898
[2] Yuri Y. Sautin and Richard J. Johnson inNucleosides Nucleotides Nucleic Acids. Jun 2008; 27(6): 608-619.uric acid:oxidant-antioxydant pardox.
[3] Fenollar, F., Fournier, P.E., Raoult, D. (2004) Molecular detection of Coxiella burnetii in the sera of patients with Q fever endocarditis or vascular infection. J. Clin. Microbiol. 42(11), 4919-24. doi: 10.1128/JCM.42.11.
[4] Harrus, S., Perlman-Avrahami, A., Mumcuoglu, K.Y., Morick, D., Baneth, G. (2011) Molecular détection of Rickettsia massiliae, Rickettsia sibirica mongolitimonae and Rickettsia conorii israelensis in ticks from Israel. Clin Microbiol Infect 17(2), 176-80, Doi: 10.1111/j.1469-0691.2010.03224.x.
[5] Khan, M.M.T., Pyle, B.H., Camper, A.K. (2010) Specific and rapid enumeration of viable but nonculturable and viable-culturable gram-negative bacteria by using flow cytometry. Appl. Environ. Microbiol.76(15), 5088-96, Doi:10.1128/AEM.02932-09.
[6] Sentausa, E., Karkouri, El, K., Robert, C., Raoult, D., Fournier, P.-E. (2012) Genome sequence of Rickettsia conorii subsp. caspia, the agent of Astrakhan fever. J. Bacteriol. 194(17), 4763-4, Doi: 10.1128/JB.00992-12.
[7] Staggemeier, R., Bortoluzzi, M., Heck, T.M.D.S., Spilki, F.R., Almeida, S.E. de M. (2015) quantitative vs. conventional pcr for detection of human adenoviruses in water and sediment samples. Rev. Inst. Med. Trop. Sao Paulo 57(4), 299-303, Doi: 10.1590/S0036-46652015000400005.

## Revendications

1. Procédé de culture in vitro de lignée de cellules eucaryotes d'organisme pluricellulaire **caractérisé en ce que** l'on ajoute dans le milieu de culture cellulaire de l'acide urique à une concentration d'au moins 0.1 g/L et on réalise la culture sous une atmosphère d'air ambiant non supplémentée en CO₂, la multiplication des dites cellules étant apte à être améliorée par culture sous atmosphère supplémentée en CO₂ en l'absence d'acide urique dans le dit milieu de culture.

2. Procédé de culture cellulaire in vitro selon la revendication 1 **caractérisé en ce qu'**on réalise une culture intracellulaire de microorganismes dans des dites cellules en milieu de culture cellulaire contenant de l'acide urique sous une atmosphère d'air ambiant non supplémentée en CO₂.

3. Procédé selon la revendication 1 ou 2, **caractérisé en ce que** l'acide urique est mis en œuvre à une concentration d'au moins 0,4 g/L, de préférence encore de 0,4 à 1 g/L.

4. Procédé selon l'une des revendications 1 à 3, **caractérisé en ce qu'**on réalise une culture intracellulaire d'un microorganisme choisi parmi les bactéries intracellulaires strictes, les virus et les parasites.

5. Procédé selon l'une des revendications 2 à 4, **caractérisé en ce qu'**on réalise une culture intracellulaire d'un dit microorganisme dans un récipient ouvert sous une atmosphère d'air ambiant non supplémenté en CO₂ et on ferme le récipient après 24heures de culture.

6. Procédé selon l'une des revendications 2 à 5, caractérisé en ce on réalise une culture intracellulaire d'une bactérie intracellulaire stricte choisie parmi les genres *Rickettsia, Orientia, Lawsonia, Coxiella, Ehrlichia, Chlamydia, Chlamydiophila, Anaplasma* et *Neorickettsia.*

7. Procédé selon la revendication 6, **caractérisé en ce qu'**on réalise une culture intracellulaire d'une bactérie intracellulaire stricte choisie parmi *Coxiella burnetii* et une espèce *Rickettsia sp.*

8. Procédé selon l'une des revendications 2 à 5, **caractérisé en ce qu'**on réalise une culture intracellulaire d'un virus choisi parmi les familles *Poxviridae, Herpesviridae, Adenoviridae,Papillomaviridae, Polyomaviridae, Parvoviridae, Picornaviridae, Caliciviridae, Astroviridae, Flaviviridae, Togaviridae, Coronaviridae, Paramyxoviridae, Rhabdoviridae, Filoviridae, Orthomyxoviridae, Arenaviridae, Bunyaviridae, Reoviridae, Hepadnaviridae* et *Retroviridae.*

9. Procédé selon la revendication 8, **caractérisé en ce que** le virus est un adénovirus.

10. Procédé selon l'une des revendications 2 à 5, **caractérisé en ce qu'**on réalise une culture intracellulaire d'un parasite choisi parmi *Plasmodium, Apicomplexa* et *Leishmania.*

11. Procédé selon la revendication 10, **caractérisé en ce que** le dit parasite est *Plasmodium falciparum.*

12. Procédé selon l'une des revendications 1 à 9, **caractérisé en ce qu'**on réalise une culture de cellules choisies parmi les lignées 3T6, 3T3, BA886, BHK21, Caco-2, CE, CLC, CRL1439, CRL1442, DF1, DH82, EC2, EC53, HEL, Hela, Hep2, HL60, HT29, J774, Jurkat, L929, Mac Coy, MRC5, MSDHP, P388, Raw264, Tatoo, TF1, THP1, U937, VERO, MDCK, BGM, MA104, ViroE6 et CLNh11.4

13. Procédé selon la revendication 12, **caractérisé en ce que** les cellules sont choisies parmi les cellules de fibroblastes humains de la lignée HEL, les cellules fibroblastiques de souris de la lignée L929, les cellules de reins de singe de la lignée Vero et les cellules de type fibroblastes de la lignée MRC5.

14. Procédé de culture selon l'une des revendications 10 ou 11 **caractérisé en ce que** les dites cellules sont des érythrocytes humains et on réalise une culture intracellulaire d'un parasite du genre Plasmodium, de préférence *Plasmodium falciparum,* dans un milieu de culture contenant de l'acide urique sous une atmosphère non supplémentée en CO₂ et non diminuée en O₂.

15. Milieu de culture utile pour un procédé selon l'une des revendications 1 à 14 **caractérisé en ce qu'**il comprend un milieu de culture de base pour culture cellulaire, le dit milieu de culture de base étant enrichi en acide urique à une concentration d'acide urique d'au moins 0,1 g/L, le dit milieu de culture de base étant apte à permettre la culture des dites cellules eucaryotes par culture sous atmosphère supplémentée en CO₂ en l'absence d'acide urique dans le dit milieu de culture de base.

16. Milieu de culture selon la revendication 15 **caractérisé en ce qu'**il comprend une concentration d'acide urique de 0,4 à 1 g/L.

17. Milieu de culture selon la revendication 15 ou 16 **caractérisé en ce que** le dit milieu de culture de base est un milieu de culture cellulaire comprenant au moins les composants suivants :
- une pluralité des sels inorganiques choisis notamment parmi les chlorure de calcium, nitrate de calcium, nitrate ferrique, sulfate de magnésium, chlorure de potassium, bicarbonate de sodium, chlorure de sodium et phosphate de sodium, de préférence au moins 4 dits sels inorganiques;
- une pluralité d'acides aminés choisis notamment parmi les arginine, cystine, glycine, histidine, isoleucine, leucine, lysine, méthionine, phénylalanine, thréonine, tryptophane, tyrosine, valine, asparagine, aspartate, glutamine, acide glutamique, proline, sérine et valine, de préférence l'ensemble des dits acides aminés;
- une pluralité de vitamines choisies notamment parmi les choline, acide folique, inositol, niacinamide, nicotinamide (acide pantothénique, pyridoxal, pyridoxine, riboflavine, thiamine, biotine, acide aminobenzoique et cobalamine, de préférence au moins 3 dites vitamines; et
- une source d'hydrate de carbone, de préférence un sucre, notamment du glucose.

18. Milieu de culture selon la revendication 17 **caractérisé en ce que** le dit milieu de culture de base est choisi parmi les milieux MEM, DMEM, RPMI et IMDM.

19. Milieu de culture selon l'une des revendications 15 à 18 **caractérisé en ce qu'**il ne contient pas de composé antioxydant additionnel dans le dit milieu de culture.

## Patentansprüche

1. Verfahren zur In-vitro-Kultur von eukaryotischen Zelllinien von mehrzelligen Organismen, **dadurch gekennzeichnet, dass** dem Zellkulturmedium Harnsäure mit einer Konzentration von mindestens 0,1 g/l beigemengt wird und die Kultur unter einer nicht mit CO₂ angereicherten Umgebungsluftatmosphäre ausgeführt wird, wobei die Vermehrung der Zellen geeignet ist, durch Kultur unter mit CO₂ angereicherter Atmosphäre bei Nichtvorhandensein von Harnsäure in dem Kulturmedium verbessert zu werden.

2. Verfahren zur In-Vitro-Zellkultur nach Anspruch 1, **dadurch gekennzeichnet, dass** eine intrazelluläre Kultur von Mikroorganismen in den Zellen im Zellkulturmedium ausgeführt wird, das Harnsäure unter einer nicht mit CO₂ angereicherten Umgebungsluftatmosphäre enthält.

3. Verfahren nach Anspruch 1 oder 2, **dadurch gekennzeichnet, dass** die Harnsäure mit einer Konzentration von mindestens 0,4 g/l, mehr zu bevorzugen von 0,4 bis 1 g/l, eingesetzt wird.

4. Verfahren nach einem der Ansprüche 1 bis 3, **dadurch gekennzeichnet, dass** eine intrazelluläre Kultur eines Mikroorganismus ausgeführt wird, der aus strikten intrazellulären Bakterien, Viren und Parasiten ausgewählt wird.

5. Verfahren nach einem der Ansprüche 2 bis 4, **dadurch gekennzeichnet, dass** eine intrazelluläre Kultur eines Mikroorganismus in einem offenen Behälter unter einer nicht mit CO₂ angereicherten Umgebungsluftatmosphäre ausgeführt wird und der Behälter nach 24-stündiger Kultur geschlossen wird.

6. Verfahren nach einem der Ansprüche 2 bis 5, **dadurch gekennzeichnet, dass** eine intrazelluläre Kultur einer strikten intrazellulären Bakterie ausgeführt wird, die ausgewählt wird aus den Gattungen *Rickettsia, Orientia, Lawsonia, Coxiella, Ehrlichia, Chlamydia, Chlamydiophila, Anaplasma* und *Neorickettsia.*

7. Verfahren nach Anspruch 6, **dadurch gekennzeichnet, dass** eine intrazelluläre Kultur einer strikten intrazellulären Bakterie ausgeführt wird, die aus *Coxiella burnetii* und einer *Rickettsia sp.* Art ausgewählt wird.

8. Verfahren nach einem der Ansprüche 2 bis 5, **dadurch gekennzeichnet, dass** eine intrazelluläre Kultur eines Virus ausgeführt wird, das aus den Familien *Poxyviridae, Herpesviridae, Adenoviridae, Papillomaviridae, Polyomaviridae, Parvoviridae, Picornaviridae, Caliciviridae, Astroviridae, Flaviviridae, Togaviridae, Coronaviridae, Paramyxoviridae, Rhabdoviridae, Filoviridae, Orthomyxoviridae, Arenaviridae, Bunyaviridae, Reoviridae, Hepadnaviridae* und *Retroviridae* ausgewählt wird.

9. Verfahren nach Anspruch 8, **dadurch gekennzeichnet, dass** das Virus ein Adenovirus ist.

10. Verfahren nach einem der Ansprüche 2 bis 5, **dadurch gekennzeichnet, dass** eine intrazelluläre Kultur eines Parasiten ausgeführt wird, der aus *Plasmodium, Apicomplexa* und *Leishmania* ausgewählt wird.

11. Verfahren nach Anspruch 10, **dadurch gekennzeichnet, dass** der Parasit *Plasmodium falciparum* ist.

12. Verfahren nach einem der Ansprüche 1 bis 9, **dadurch gekennzeichnet, dass** eine Kultur von Zellen ausgeführt wird, die aus den Linien 3T6, 3T3, BA886, BHK21, Caco-2, CE, CLC, CRL1439, CRL1442, DF1, DH82, EC2, EC53, HEL, Hela, Hep2, HL60, HT29, J774, Jurkat, L929, Mac Coy, MRC5, MSDHP, P388, Raw264, Tatoo, TF1, THP1, U937, VERO, MDCK, BGM, MA104, ViroE6 und CLNh11.4 ausgewählt werden.

13. Verfahren nach Anspruch 12, **dadurch gekennzeichnet, dass** die Zellen aus humanen fibroblastischen Zellen der Linie HEL, fibroblastischen Zellen von Mäusen der Linie 929, Affennierenzellen der Linie Vero und Zellen vom Typ Fibroblasten der Linie MRC5 ausgewählt werden.

14. Kulturverfahren nach einem der Ansprüche 10 oder 11, **dadurch gekennzeichnet, dass** die Zellen humane Erythrozyten sind und eine intrazelluläre Kultur eines Parasiten der Gattung Plasmodium, vorzugsweise *Plasmodium falciparum,* in einem Kulturmedium ausgeführt wird, das Harnsäure unter einer nicht mit CO₂ angereicherten und nicht an O₂ abgereicherten Atmosphäre enthält.

15. Kulturmedium für ein Verfahren nach einem der Ansprüche 1 bis 14, **dadurch gekennzeichnet, dass** es ein Basiskulturmedium zur Zellkultur umfasst, wobei das Basiskulturmedium mit Harnsäure mit einer Harnsäurekonzentration von mindestens 0,1 g/l angereichert ist, wobei das Basiskulturmedium geeignet ist, die Kultur der eukaryotischen Zellen durch Kultur unter mit CO₂ angereicherter Atmosphäre bei Nichtvorhandensein von Harnsäure in dem Basiskulturmedium zu ermöglichen.

16. Kulturmedium nach Anspruch 15, **dadurch gekennzeichnet, dass** es eine Harnsäurekonzentration von 0,4 bis 1 g/l umfasst.

17. Kulturmedium nach Anspruch 15 oder 16, **dadurch gekennzeichnet, dass** das Basiskulturmedium ein Zellkulturmedium ist, das mindestens die folgenden Bestandteile umfasst:
- eine Vielzahl von anorganischen Salzen, die insbesondere unter Calciumchlorid, Calciumnitrat, Eisennitrat, Magnesiumsulfat, Kaliumchlorid, Natriumbicarbonat, Natriumchlorid und Natriumphosphat, vorzugsweise mindestens 4 der anorganischen Salze, ausgewählt ist;
- eine Vielzahl von Aminosäuren, die insbesondere unter Arginin, Cystin, Glycin, Histidin, Isoleucin, Leucin, Lysin, Methionin, Phenylalanin, Threonin, Tryptophan, Tyrosin, Valin, Asparagin, Aspartat, Glutamin, Glutaminsäure, Prolin, Serin und Valin, vorzugsweise sämtlichen dieser Aminosäuren, ausgewählt sind;
- eine Vielzahl von Vitaminen, die insbesondere aus Cholin, Folsäure, Inositol, Niacinamid, Nicotinamid, Pantothensäure, Pyridoxal, Pyridoxin, Riboflavin, Thiamin, Biotin, Aminobenzoesäure und Cobalamin, vorzugsweise mindestens 3 dieser Vitamine, ausgewählt sind; und
- eine Kohlenhydratquelle, vorzugsweise ein Zucker, insbesondere Glucose.

18. Kulturmedium nach Anspruch 17, **dadurch gekennzeichnet, dass** das Basiskulturmedium aus den Medien MEM, DMEM, RPMI und IMDM ausgewählt ist.

19. Kulturmedium nach einem der Ansprüche 15 bis 18, **dadurch gekennzeichnet, dass** es keine zusätzliche antioxidantische Verbindung in dem Kulturmedium enthält.

## Claims

1. Method for in vitro culture of a eukaryotic cell line of a multicellular organism **characterized in that** uric acid is added into the cell culture medium at a concentration of at least 0.1 g/L and the culture is performed under an ambient air atmosphere not supplemented with CO₂, the multiplication of said cells being able to be improved by culture under atmosphere supplemented with CO₂ in the absence of uric acid in said culture medium.

2. Method for in vitro cell culture according to claim 1, **characterized in that** an intracellular culture of microorganisms is performed in said cells in cell culture medium containing uric acid in an ambient air atmosphere not supplemented with CO₂.

3. Method according to claim 1 or 2, **characterized in that** uric acid is implemented at a concentration of at least 0.4 g/L, more preferably 0.4 to 1 g/L.

4. Method according to one of claims 1 to 3, **characterized in that** an intracellular culture is performed of a microorganism chosen from among obligate intracellular bacteria, viruses and parasites.

5. Method according to one of claims 2 to 4, **characterized in that** an intracellular culture is performed of said microorganism in an open container under an ambient air atmosphere not supplemented with CO₂ and the container is closed after 24 hours of culture.

6. Method according to one of claims 2 to 5, **characterized in that** an intracellular culture is performed of an obligate intracellular bacteria chosen from among the genera *Rickettsia, Orientia, Lawsonia, Coxiella, Ehrlichia, Chlamydia, Chlamydophila, Anaplasma* and *Neorickettsia.*

7. Method according to claim 6, **characterized in that** an intracellular culture is performed of an obligate intracellular bacteria chosen from among *Coxiella burnetii* and a *Rickettsia spp.* species.

8. Method according to one of claims 2 to 5, **characterized in that** an intracellular culture is performed of a virus chosen from among the families *Poxviridae, Herpesviridae, Adenoviridae, Papillomaviridae, Polyomaviridae, Parvoviridae, Picornaviridae, Caliciviridae, Astroviridae, Flaviviridae, Togaviridae, Coronaviridae, Paramyxoviridae, Rhabdoviridae, Filoviridae, Orthomyxoviridae, Arenaviridae, Bunyaviridae, Reoviridae, Hepadnaviridae* and *Retroviridae.*

9. Method according to claim 8, **characterized in that** the virus is an adenovirus.

10. Method according to one of claims 2 to 5, **characterized in that** an intracellular culture is performed of a parasite chosen from among *Plasmodium, Apicomplexa* and *Leishmania.*

11. Method according to claim 10, **characterized in that** said parasite is *Plasmodium falciparum.*

12. Method according to one of claims 1 to 9, **characterized in that** a culture is performed of cells chosen from among the lines 3T6, 3T3, BA886, BHK21, Caco-2, CE, CLC, CRL1439, CRL1442, DF1, DH82, EC2, EC53, HEL, Hela, Hep2, HL60, HT29, J774, Jurkat, L929, Mac Coy, MRCS, MSDHP, P388, Raw264, Tatoo, TF1, THP1, U937, VERO, MDCK, BGM, MA104, ViroE6 and CLNh11.4

13. Method according to claim 12, **characterized in that** the cells are chosen from among human fibroblasts of the HEL line, mouse fibroblastic cells of the L929 line, monkey kidney cells of the Vero line and fibroblast type cells of the MRCS line.

14. Culture method according to one of claims 10 or 11, **characterized in that** said cells are human erythrocytes and an intracellular culture is performed of a parasite of the Plasmodium genus, preferably *Plasmodium falciparum,* in a culture medium containing uric acid in an atmosphere not supplemented with CO₂ and not reduced in O₂.

15. Culture medium useful for a method according to one of claims 1 to 14, **characterized in that** it comprises a basic culture medium for cell culture, said basic culture medium being enriched in uric acid at a uric acid concentration of at least 0.1 g/L, said basic culture medium being able to allow culture of said eukaryotic cells by culture under atmosphere supplemented with CO₂ in the absence of uric acid in said basic culture medium.

16. Culture medium according to claim 15, **characterized in that** it comprises a uric acid concentration of 0.4 to 1 g/L.

17. Culture medium according to claim 15 or 16, **characterized in that** said basic culture medium is a cell culture medium comprising at least the following components:
- a plurality of inorganic salts chosen especially from among calcium chloride, calcium nitrate, ferric nitrate, magnesium sulfate, potassium chloride, sodium bicarbonate, sodium chloride and sodium phosphate, preferably at least 4 of said inorganic salts;
- a plurality of amino acids chosen especially from among arginine, cystine, glycine, histidine, isoleucine, leucine, lysine, methionine, phenylalanine, threonine, tryptophane, tyrosine, valine, asparagine, aspartate, glutamine, glutamic acid, proline, serine and valine, preferably all of said amino acids;
- a plurality of vitamins especially chosen from among choline, folic acid, inositol, niacinamide, nicotinamide (pantothenic acid, pyridoxal, pyridoxin, riboflavin, thiamine, biotin, aminobenzoic acid and cobalamine, preferably at least 3 of said vitamins; and
- a carbohydrate source, preferably a sugar, especially glucose.

18. Culture medium according to claim 17 **characterized in that** said basic culture medium is chosen from among MEM, DMEM, RPMI and IMDM media.

19. Culture medium according to one of claims 15 to 18, **characterized in that** it does not contain an additional antioxidant compound in said culture medium.
